# EUROPÄISCHE PATENTSCHRIFT

(11) **EP 3 554 756 B1**
(45) Veröffentlichungstag und Bekanntmachung des Hinweises auf die Patenterteilung: **27.09.2023**
(21) Anmeldenummer: 17801008.8
(22) Anmeldetag: 12.10.2017
(51) Int. Cl.: B23K 26/36, B29D 11/00, G02B 1/04, B23K 26/082, B23K 26/0622, B33Y 80/00, B23K 103/00, B22F 3/105

(54) **VERFAHREN ZUR HERSTELLUNG EINER TRANSMITIVEN OPTIK**
METHOD FOR PRODUCING A TRANSMISSIVE OPTICS
PROCÉDÉ DE FABRICATION D'UNE OPTIQUE À TRANSMISSION

(30) Priorität: 13.12.2016 DE 102016014747; 28.03.2017 DE 102017002986
(43) Veröffentlichungstag der Anmeldung: 23.10.2019
(73) Patentinhaber: Aixlens GmbH, 52477 Alsdorf (DE)
(72) Erfinder: VON WALLFELD, Axel, 52134 Herzogenrath (DE); POPRAWE, Reinhart, 52074 Aachen (DE); FORNAROLI, Christian, 8582 Dozwil (CH); WILLENBORG, Edgar, 52159 Roetgen-Rott (DE); WEINGARTEN, Christian, 52064 Aachen (DE); CLASEN, Uwe, 52072 Aachen (DE)
(74) Vertreter: Castell, Klaus
(86) Internationale Anmeldenummer: PCT/DE2017/000337
(87) Internationale Veröffentlichungsnummer: WO 2018/108192

(56) Entgegenhaltungen:
- WO-A1-2004/026566
- WO-A1-2012/119761
- WO-A1-2015/165435
- US-A1- 2015 146 162
- US-A1- 2015 167 926

## Beschreibung

Die Erfindung betrifft ein Verfahren zur Herstellung einer transmitiven Optik aus einem Rohling. Dabei ist eine transmitive Optik in der Regel eine Linse. Die Erfindung betrifft insbesondere die Herstellung einer Intraokularlinse, einer Kontaktlinse, eines refraktiven Implantats oder eines Brillenglases. Ferner betrifft die Erfindung auch die Bearbeitung von optischen Oberflächen, die teils verspiegelt und teils strahlungsdurchlässig sind.

Die Behandlung beliebiger 3D-Formflächen mittels eines Lasers wird in der WO 96/31315 beschrieben. Hierbei geht es insbesondere um die Herstellung von präzisen Dichtflächen an Formwerkzeugen.

Die WO 2012/119761 A1 betrifft ein Verfahren zur Fertigung optischer Elemente durch eine Bearbeitung mit energetischer Strahlung, vorzugsweise mit Laserstrahlung. Hierbei werden Rohlinge aus Quarzglas zunächst durch einen Grobabtrag und anschließend durch Polieren und einen Feinstabtrag behandelt. Dieses iterative Verfahren mit mehreren Verfahrensschritten eignet sich vor allem für harte Materialien, wie Glas oder Stahl.

Die DE 10 2007 058 105 A1 beschreibt ein Verfahren zur Herstellung einer transmittiven Optik mit einem Abtragslaser. Bei diesem Verfahren wird der Materialabtrag aber nicht mit dem Laser an einem Rohling erzielt, sondern mit einem Abtragsmittel, das separat mit einem Verdampfer der zu bearbeitenden Fläche zugeführt wird. Bei diesem Verfahren wird somit der Materialabtrag mit dem Abtragsmittel erzielt und der Laser wirkt nicht auf die Oberfläche des Rohlings sondern auf das Abtragsmittel, das als dampfförmige Phase oder in kondensierter Flüssigphase zwischen dem Laser und dem Rohling die Laserstrahlung absorbiert. Die in dieser Anmeldung angegebenen Pulsdauern unter 500 fs beziehen sich auf die Laserstrahlung, die auf das dampfförmige bzw. flüssige Abtragsmittel trifft und nicht auf einen Laserstrahl, der zu einem Materialabtrag am Rohling führt. Derartige Verfahren eignen sich für besonders harte Materialien.

Die US 5 143 660 A beschreibt einen Spritzgussprozess zur Herstellung von Kunststofflinsen. Dabei werden in der Linse spezielle Löcher ausgebildet, die der Aufnahme von Flüssigkeiten wie insbesondere von Medikamenten dienen.

Die WO 2015 165435 A1 (Grundlage für den Oberbegriff des Anspruches 1) zeigt ein gattungsgemäßes Verfahren, das jedoch für einen industriellen Einsatz nicht zu verwenden ist.

Der Erfindung liegt daher die Aufgabe zugrunde, ein gattungsgemäßes Verfahren vorzustellen, das eine schnelle kostengünstige Herstellung ermöglicht.

Diese Aufgabe wird mit einem Verfahren mit den Merkmalen des Patentanspruches 1 gelöst. Vorteilhafte Weiterbildungen sind Gegenstand der Unteransprüche.

Die extrem kleine Pulsdauer des Abtragslasers hat zur Folge, dass das während des Pulses verdampfende Material des Rohlings nach der Verdampfung den Energieeintrag nicht behindert und nach jedem Puls, innerhalb der kurzen Unterbrechung zwischen zwei Pulsen, der Dampf auch aus der Bearbeitungszone weitestgehend entweichen kann oder der Laser auf eine andere Bearbeitungszone gerichtet werden kann. Dies ermöglicht eine präzise Oberflächenbearbeitung, bei der auf der Oberfläche des Rohlings sehr eng aneinander liegende kleine Krater erzeugt werden können.

Die kurze Pulsdauer führt dazu, dass nahezu keine Interaktion zwischen Schmelze oder unter Laserstrahlung verdampfendem Material des Rohlings eintritt. Der Materialabtrag wird durch direkte Verdampfung erzielt und dadurch wird das behandelte Material kaum geschädigt. Die ultrakurzen Laserpulse mit Dauern von einigen Femtosekunden bis hin zu einigen Piko- oder Nanosekunden erlauben neue Bearbeitungsverfahren, die mit konventionellen Werkzeugen nicht möglich sind. Diese Laserblitze führen zu extremen hohen Spitzenintensitäten, die aufgrund der starken zeitlichen Kompression bereits mit relativ geringen Impulsenergien erreicht werden können. Dies ermöglicht einen hoch präzisen Materialabtrag und die Bearbeitung temperartursensibler Materialien.

Erfindungsgemäß ist der Rohling aus Kunststoff hergestellt. Hierfür eignen sich Monomere und Polymere und auch Kombinationen hieraus und insbesondere transparente Kunststoffe.

Besonders bevorzugt weist der Rohling ein Acrylat auf. Dabei kann der Rohling auch aus unterschiedlichen Kunstsoffen hergestellt sein. Das Acrylat ist vorzugsweise transparent oder teilweise transparent. Es können Acrylate mit oder ohne Weichmacher verwendet werden. Geeignete Materialien sind beispielsweise HI56 SPECS^{®}, PMMA, CI26^{®} oder Contaflex CI18^{®}, Hydrogel, Silikon oder auch Kombinationen aus einem Collagen und einem Polymer, wie beispielsweise Collamer^{®}.

Der Rohling kann von einem Grundkörper, wie insbesondere einer zylinderförmigen Stange oder einer Platte, abgeschnitten oder ausgeschnitten werden. Besonders vorteilhaft ist es, wenn der Rohling durch Spritzgießen oder Extrudieren hergestellt wird.

Eine besonders vorteilhafte Ausführungsvariante sieht vor, dass der Rohling durch additive Fertigung (3D-Druck) aus einem pulverförmigen, flüssigen oder gasförmigen Material hergestellt ist. Dabei kann der Rohling bereits eine an die Endform angepasste dreidimensionale Form aufweisen, die auch asymmetrisch ausgebildet sein kann. Sowohl das aufbauende Verfahren als auch das Kombinieren mit dem beschriebenen Abtragsverfahren können hier angewandt werden.

Eine vorteilhafte Ausführungsvariante sieht vor, dass ein Rohling verwendet wird, der einen Dichtegradienten aufweist. Ein derartiger Dichtegradient führt innerhalb des Rohlings zu einem örtlich unterschiedlichen optischen Brechungsverhalten. Dies führt dazu, dass durch die Einstellung der optischen Dichte innerhalb des Rohlings ein Rohling hergestellt und verwendet werden kann, der mit einer geringeren Oberflächenkrümmung die gleiche Lichtbrechung wie ein homogener Rohling bewirkt.

Alternativ oder kumulativ wird vorgeschlagen, dass der Rohling aus unterschiedlichen Materialien oder Materialarten hergestellt ist. Dadurch entsteht ein Multimaterialrohling, der durch die Materialauswahl und die örtliche Materialanordnung zu einem bestimmten Brechungsverhalten führt. Auch in Folge einer bestimmten Auswahl von Materialien mit unterschiedlichen Dichten kann ein Dichtegradient erzielt werden, der das Brechungsverhalten des Rohlings und der Optik bestimmt.

Wenn beispielsweise bei einem Zylinder die äußeren Mantelbereiche eine andere optische Dichte aufweisen als der Kernbereich, wird eine auf die ebene Seite des Zylinders auftreffende Strahlung entsprechend der optischen Dichte des Materials abgelenkt, ohne dass die Oberfläche gekrümmt ausgebildet sein muss. Dadurch ermöglicht der Dichtegradient bei einer kreisförmigen Linse in radialer Richtung eine Verringerung der Krümmung der Oberfläche. Dies hat zur Folge, dass die Bearbeitung der weniger gekrümmten Oberfläche mit einem Laserstrahl erleichtert wird, da in Folge der geringeren Krümmung der Laserstrahl leichter annähernd im rechten Winkel zu einer Normalen an der Krümmung der Oberfläche geführt werden kann. Der Rohling kann jedoch auch bereits ein Brechungsverhalten aufweisen, das an die gewünschte Soll-Form der Linse angenähert ist. Dadurch wird der Bearbeitungsaufwand der Linse reduziert.

Daher wird weiterbildend vorgeschlagen, dass ein im Querschnitt kreisrunder Rohling verwendet wird, der zur Mitte hin eine andere optische Dichte aufweist als zum Rand hin.

Ein Dichtegradient an der Linse ermöglicht es, die Linse mit einer geringeren Krümmung oder sogar ohne Krümmung herzustellen. Dies führ zu einer Linse, die flexibel und aufrollbar herstellbar ist. Dadurch wird es möglich, eine Linse sehr klein zu falten oder zusammenzurollen, um sie durch eine besonders kleine Öffnung ins Auge einzuführen. Somit reicht ein sehr kleiner Schnitt an der Hornhaut, um die alte zerstörbare Linse herauszunehmen und eine neue Linse ins Auge einzuführen, die sich dann erst im Auge entfaltet oder ausrollt.

Die Ausführungen zu den unterschiedlichen Materialien und dem Dichtegradienten sind jede für sich auch unabhängig von der im Patentanspruch 1 angegebenen Pulsdauer erfindungswesentlich.

Vorteilhaft ist es, wenn die Pulsenergie während des Abtrages und/oder währende des Polierens variiert wird. Somit wird vorgeschlagen, dass die Energie des Lasers zeitlich variiert wird. Dies ermöglicht es, in bestimmten Oberflächenbereichen mit einer höheren Pulsenergie abzutragen oder zu Polieren als in anderen Oberflächenbereichen. Wenn der Laserstrahl beispielsweise auf einer mäandrierenden Linie über die Oberfläche des Rohlings geführt wird, kann beispielsweise in den Umlenkbereichen der Energieeintrag verringert werden.

Sofern das Abtragen von Materie im Vordergrund steht, um die Form des Rohlings zu verändern, spricht man von einem Abtragen bzw. von einem Abtragslaser. Sofern hingegen die Form möglichst unverändert bleiben soll und nur die Oberfläche geglättet werden soll spricht man von Polieren bzw. von einem Polierlaser.

Besonders vorteilhaft ist es, wenn nach jedem Laserpuls die Form der bearbeiteten Optik im Bereich der bearbeiteten Oberfläche gemessen wird. Dabei kann entweder der Gesamtabtrag pro Flächenbereich ermittelt werden oder es kann sogar die Kraterform ermittelt werden. Hierzu dient bevorzugt eine optische Kohärenztomographie. Dies ermöglicht es, die Ortsdaten zu speichern und bei einer anschließenden Bearbeitung der Fläche oder der Bearbeitung einer angrenzenden Fläche diese Ortsdaten zu berücksichtigen. Dabei wird entweder direkt nach einem Puls gemessen oder in der Zeit bis zur wiederholten Bearbeitung der vermessenen Fläche oder bis zur Bearbeitung einer angrenzenden Fläche. Dies ermöglicht eine Präzision im Mikrometerbereich oder sogar im Submikrometerbereich.

Neben der Vermessung der bearbeiteten Oberfläche ist es vorteilhaft, wenn während der Bearbeitung die Prozesstemperatur mit einem Messsystem, vorzugsweise mit einem Pyrometer oder einer Thermokamera überwacht wird. Dies ermöglicht es, auch die Prozesstemperatur zu regeln, um sie in einer definierten Bandbreite zu halten.

Der Laserpuls hat in der Regel über der Fläche eine gaußsche Intensitätsverteilung. Eine besondere Präzision bei der Bearbeitung von Oberflächen wird jedoch dadurch erzielt, dass die Pulsenergieverteilung eines Pulses örtlich asymmetrisch ist. In Abweichung von einem Rechteckimpuls können somit die Flanken des Rechtecks unterschiedlich ausgebildet sein. Insbesondere kann der Puls an einer Seite zur Mitte hin stärker ansteigen als er auf der anderen Seite von der Mitte weg abfällt. Dadurch kann die Energieverteilung über der Laserstrahlfläche derart variiert werden, dass beim Auftreffen des Strahls auf einer gekrümmten Fläche eine an die gekrümmte Fläche angepasste Verdampfung erzielt wird. Insbesondere kann auch die Tiefe des durch den Laserpuls erzeugten Kraters verringert werden, ohne dass während des Pulses das verdampfte Volumen reduziert wird.

Dies ermöglicht einen nahezu konstanten Abtrag über der zu behandelnden Fläche. Außerdem kann das Abtragsprofil beliebig variiert und manipuliert werden.

Außerdem sollte auch über die gesamte zu bearbeitende Fläche die Kratertiefe im Wesentlichen konstant sein. Im Wesentlichen heißt hierbei, dass die Intensitätsverteilung des Laserstrahls so eingestellt ist, dass beispielsweise bei einer Bestrahlung einer Kreisfläche mit einem Radius r die durchschnittliche Kratertiefe auf einer Kreisfläche mit einem Radius r/2 maximal doppelt so tief ist wie die durchschnittliche Kratertiefe in einer Ringfläche im Bereich r/2 bis r. Vorzugsweise liegt die Abweichung sogar im sub-µm-Bereich.

Eine Ausführungsform sieht vor, dass die Pulsenergieverteilung während eines Pulses auf einer kreisförmigen oder einer ovalen Fläche in radialer Richtung mindestens ein Maximum aufweist. Die Intensitätsverteilung des Pulses kann beispielsweise als Form eines Vulkankraters abgebildet werden, sodass in den Randbereichen der kreisförmigen ovalen Fläche eine höhere Intensität wirkt als im zentralen Bereich. Dies ermöglicht es, je nach Aufgabenstellung, die Intensitätsverteilung des Pulses über der vom Puls getroffenen Fläche individuell zu variieren. Diese Pulsform kann einmalig eingestellt werden oder während der Bearbeitung verändert werden. Daher wird vorgeschlagen, dass während der Bearbeitung die Pulsenergieverteilung quer zur Strahlungsrichtung variiert wird.

Die Ausführungen zur örtlichen und zur zeitlichen Energieverteilung sind jede für sich auch unabhängig von der im Patentanspruch 1 angegebenen Pulsdauer erfindungswesentlich.

Die Auswirkung eines Laserstrahls ist unterschiedlich, je nachdem, ob der Laserstrahl senkrecht oder in einem Winkel auf die zu bearbeitende Fläche trifft. Dies führt dazu, dass auch die Ausrichtung des Laserstrahls relativ zur bearbeiteten Oberfläche das Abtragsergebnis beeinflusst. Um diese Unterschiede einzuschränken wird vorgeschlagen, dass während der Bearbeitung einer gekrümmten Optik die Ausrichtung des Laserstrahls im Wesentlichen senkrecht zu einer Tangentenebene im Schnittpunkt von Laserstrahl und Optik gehalten wird. Im Wesentlichen heißt hier: bei einer Abweichung von weniger als 40 % und bevorzugt weniger als 10 %. Da senkrecht einen Winkel des Laserstrahls von 90° zur Tangentenebene beschreibt, beschreibt im Wesentlichen senkrecht beispielsweise einen Winkel des Laserstrahls zur Tangentenebene von größer 70°. Alternativ oder kumulativ kann auch die Intensität oder die Intensitätsverteilung des Laserstrahls je nach Auftrittswinkel variiert werden.

Um den Laserstrahl möglichst im rechten Winkel auf die zu bearbeitende Fläche auftreffen zu lassen, kann der Strahl über Spiegel abgelenkt werden. Es können jedoch auch die Positionen des Lasers und der zu bearbeitenden Optik relativ zueinander verändert werden. Daher wird vorgeschlagen, dass während der Bearbeitung die Position und/oder die Ausrichtung des Rohlings oder des Lasers bewegt werden.

Der Bearbeitungsaufwand kann dadurch stark reduziert werden, dass ein Rohling verwendet wird, der bereits auf einer Seite oder mehreren Seiten eine definierte Form, wie insbesondere eine konvexe oder konkave Form aufweist und nur noch auf einer Seite mit dem Laser bearbeitet wird. Eine bevorzugte Ausführungsvariante sieht vor, dass der Rohling auf einer Seite symmetrisch geformt ist und auf einer anderen Seite asymmetrisch oder als Freiform bearbeitet wird.

Die Ausführungen zur Ausrichtung des Laserstrahls und zur Form des Rohlings sind jede für sich auch unabhängig von der im Patentanspruch 1 angegebenen Pulsdauer erfindungswesentlich

Die präzise Bearbeitung von Optiken ermöglicht es, für einen Patienten individuell eine Sehhilfe wie insbesondere eine Intraokularlinse oder eine Kontaktlinse zu fertigen. Hierfür wird vorgeschlagen, dass zunächst ein Auge eines Patienten vermessen wird und damit ein Datensatz erzeugt wird und anschließend auf der Grundlage der Daten dieses Datensatzes der Abtragslaser und/oder der Polierlaser gesteuert wird.

Dabei kann das Auge mittels Biometrie und/oder Topometrie zur Ermittlung der Achsenlänge des Augapfels, der vorderen Hornhautoberfläche, der hinteren Hornhautoberfläche der Hornhautdicke und/oder des Brechungsindex der Hornhaut vermessen werden. Die Topometrie erlaubt es, mit einem Ophthalmometer oder einem Keratometer neben den Zentralradien auch die peripheren Hornhautradien zu erfassen, um aussagekräftige Flächenparameter beispielsweise für die Anpassung von Kontaktlinsen oder Intraokularlinsen zu gewinnen. Die Topometrie liefert Einzelmesswerte, aus denen man näherungsweise auf den Flächencharakter schließen kann, und die Keratographie liefert ein komplettes Flächenprofil. Daraus ergibt sich ein Datensatz für die Soll-Form, der eine individuelle Herstellung einer Linse oder einer anderen Sehhilfe ermöglicht. Dabei können die Sehfehler von allen Elementen des Auges stammen. Eine Vermessung der einzelnen Elemente oder mittels Ray-Tracing zur Ermittlung der Beugung eines durch ein Auge verlaufenden Strahlungsbündels erlauben es, eine Sehkorrektur zu definieren, um mit einem Sehkorrekturmittel diese Fehler auszugleichen.

Vorteilhaft ist es, wenn bei der Vermessung bereits ein Datensatz erzeugt wird, der auf einfache Art und Weise in ein Computersteuerprogramm zur Fertigung einer Linse umgesetzt werden kann.

Es können verschiedene individuell hergestellte Sehhilfen oder Sehkorrekturmittel verwendet werden oder es können Standardsehhilfen mit individuell hergestellten Sehkorrekturmitteln kombiniert werden. Es ist jedoch auch möglich, nur die Linse durch eine korrigierte Intraokularlinse zu ersetzen, um möglichst weitgehend alle Fehler des Auges zu korrigieren.

Ein derartiges Verfahren wurde in der EP 0 954 255 B1 beschrieben. Es wurde vorgeschlagen, eine künstliche Linse mit einem Laser zu beschneiden. Dieses Verfahren ist wirtschaftlich nicht realisierbar, da es aufwändig ist, eine Linse mit einem Laser so zu beschneiden, dass sie genau einer speziellen Sollform entspricht und so glatt ist, dass keine ungewollten Lichtbrechungen entstehen. Erst die Kombination des Verfahrens mit einem Kunststoffmaterial wie insbesondere einem Acrylat und einem Kunststoff verdampfenden Laser führte zu einem wirtschaftlichen Verfahren. Dieses Verfahren ist insbesondere als zweistufiges Verfahren mit einem für einen Materialabtrag verwendeten Abtragslaser und einem für einen Polierschritt verwendeten Polierlaser auch unabhängig von den zuvor genannten Verfahrensschritten erfindungswesentlich.

Dabei kann die Linse auch multifokale Funktionen aufweisen. Außerdem können mit der Linse auch Fehler durch Streuung im Auge, wie insbesondere altersbedingte Streuzentren im Glaskörper korrigiert werden. Außerdem können Reflektionen im Auge, lokale Absorptionen, Veränderungen der Polarisation im Auge und individuelle Sehschwächen derart korrigiert werden, dass die Qualität der Korrektur die optische Auflösung der Netzhaut erreicht oder sogar übersteigt (Retina-Quality-IOL).

Weiterhin wird vorgeschlagen, durch das Abtragen und/oder Polieren die optische Dichte der Oberfläche der Optik gezielt derart zu verändern, dass der veränderte Brechungsindex Reflexe verhindert. Insbesondere durch ein extremes Polieren können Lichtbrechungen, wie Fabry-Pérot-Effekt und Mehrfachreflexion, minimiert werden. Die Politur wirkt dann wie ein Anti-Relex-Coating. Dies kann insbesondere auch dadurch erzielt werden, dass in unterschiedlichen Schichten der Brechungsindex verändert wird.

Insbesondere um Streustrahlung zu eliminieren und um Linsen mit vergrößerter Tiefenschärfe und Multifocal-IOLs herstellen zu können wird vorgeschlagen, mit der Laserstrahlung das Material des Rohlings derart zu verändern, dass die fertige Linse einen optischen Dichtegradienten aufweist. Eine Veränderung des Dichtegradienten kann durch abtragende und additive Verfahren erzielt werden. Beispielsweise kann durch eine radial mitgedrehte Orientierung des Laserlichtflecks oder des Laserfokus ein Dichtegradient erzielt werden. Insbesondere kann ein Dichtegradient auch durch eine definierte Strahlverteilung erzielt werden. So kann in einem radial inneren Bereich mit einer höheren Intensität des Laserstrahls gearbeitet werden als in einem radial weiter außen liegenden Bereich um einen Dichtegradienten oder Bereiche unterschiedlicher Dichte zu erzielen. Entsprechend kann auch in einem radial inneren Bereich mit einer niedrigeren Intensität des Laserstrahls gearbeitet werden als in einem radial weiter außen liegenden Bereich um einen Dichtegradienten oder Bereiche unterschiedlicher Dichte zu erzielen. Dieser Gradient oder Dichteunterschied kann bei der Berechnung des Brechungsverhaltens einer transmitiven Optik berücksichtigt werden. Vorteilhaft ist ein optischer Dichteunterschied zwischen zwei Bereichen der Linse von mindestens 0,01. Somit können beliebige, vorzugsweise radial symmetrische, Brechungsindexgradienten als Dichtesprung oder als kontinuierlicher Dichteübergang erzeugt werden.

Ein besonders wesentliches Einsatzgebiet des Verfahrens liegt daher in der Herstellung einer Optik einer Intraokularlinse.

Erfindungsgemäß wird das Verfahren so betrieben, dass er einen Materialabtrag von 0,02 µm bis 5 µm und besonders bevorzugt von 0,02 µm bis 0,5 µm pro Puls bewirkt. Je nach gefordertem Materialabtrag kann der Abtrag auch in mehreren Schichten durchgeführt werden, wobei der Abtrag pro Schicht kleiner als 2 µm, besonders bevorzugt kleiner als 1 µm sein sollte. Dabei kann zunächst mit einem größeren Abtrag gearbeitet werden und bei Annäherung an die Soll-Form kann der Abtrag pro Schicht reduziert werden. Dies führt dazu, dass zunächst mit größeren Intensitäten oder auch größeren bestrahlten Flächen ein größerer Abtrag bewirkt wird und anschließend ein kleinerer Abtrag pro Puls bewirkt wird, damit die Oberfläche möglichst glatt wird und wenig poliert werden muss.

Es hat sich herausgestellt, dass es vorteilhaft ist, wenn der Abtragslaser mit einer Laserwellenlänge von 100 bis 1200 nm und vorzugsweise kleiner 400 nm, wie insbesondere zwischen 193 nm und 370 nm betrieben wird. Bevorzugte Wellenlängen sind 193, 248, 266, 343 und 355 nm.

Erfindungsgemäß liegt während des Abtrags der Fokusdurchmesser des Abtragslasers zwischen 5 und 50 µm und vorzugsweise bei etwa 20 µm.

Erfindungsgemäß liegt die Scangeschwindigkeit des Abtragslasers bei 100 bis 5000 mm/s und vorzugsweise bei 500 bis 5000 mm/s und besonders bevorzugt bei etwa 1000 mm/s.

Erfindungsgemäß liegt die Pulsenergie des Abtragslasers bei 0,1 µJ bis 10 µJ und vorzugsweise bei etwa 1 µJ.

Die Repititionsrate des Abtragslasers kann bei 5 kHz bis 5000 kHz und vorzugsweise bei 50 bis 200 oder 10 bis 500 kHz liegen.

Eine vorteilhafte Verfahrensvariante sieht vor, dass mit dem Laserstrahl des Abtragslasers zunächst beabstandet zur Soll-Form Material entfernt wird bis mindestens 50 % des Materials entfernt sind und erst dann in einem Bereich näher an der Soll-Form Material entfernt wird. Dabei kann der Laser bergauf und bergab geführt werden. Bei einer Laserführung bergauf wird zunächst radial ganz außen nur ein Stück abgetragen und dann in der Regel ein kleineres weiter radial nach innen reichendes Stück. Bergab heißt, dass zunächst ein großes Stück von radial au-ßen bis zur Endform abgetragen wird und danach ein kleineres Stück darunter.

Außerdem hat es sich als vorteilhaft erwiesen, wenn der Abstand der durch die einzelnen Laserpulse auf der Oberfläche innerhalb einer Abtragsschicht erzeugten Abtragskrater nicht konstant ist. Zur Einstellung der mittleren abgetragenen Schichtdichte kann auch dieser Abstand variiert werden. Dadurch wird der Abtrag pro Fläche durch den Abstand der Pulsauftrittsflächen auf der zu bearbeitenden Optik verändert. Eng beieinanderliegende Pulsauftrittsflächen bewirken einen größeren Abtrag, während weit auseinanderliegende Pulsauftrittsflächen einen geringeren Abtrag bewirken.

Für das Polieren wird vorgeschlagen, dass der Polierlaser gepulst oder moduliert mit einer Pulsdauer von über 1 µs betrieben wird. Dadurch kann beispielsweise bei Kunststoffen eine optimale Politur erreicht werden.

Bevorzugte Laserwellenlängen für das Polieren liegen im Bereich zwischen 0,1 µm und 100 µm und vorzugsweise zwischen 9 µm und 11 µm oder zwischen 0,1 µm und 0,4 µm oder zwischen 1 bis 12 µm.

Eine spezielle Verfahrensführung sieht vor, dass der Polierlaser kontinuierlich betrieben wird. Der Laser wird somit während der Politur nicht gepulst, sondern der Strahl wird relativ zur Oberfläche der Optik, vorzugsweise sogar mit sich verändernder Intensität, bewegt. Im Gegensatz zu einem gepulsten Laser liegt somit immer eine gewisse Laserleistung vor.

Für das Polieren hat sich als vorteilhaft erwiesen, wenn der Polierlaser einen Strahldurchmesser am Werkstück von weniger als 10 mm und bevorzugt zwischen 0,1 mm und 8 mm aufweist. Der Poliervorgang kann dadurch vereinfacht werden, dass der Polierlaser einen Strahldurchmesser am Werkstück von größer oder gleich der zu polierenden Fläche aufweist. Damit wird es möglich, diese Fläche auf einmal zu polieren, ohne den Laser über der Fläche hin und her zu bewegen.

Eine vorteilhafte Vorschubgeschwindigkeit des Polierlasers liegt zwischen 1 und 100 mm/s.

Weiterhin ist es vorteilhaft, wenn der Polierlaser durch eine Scanbewegung mit einer Scangeschwindigkeit von 500 mm/s bis 20 000 mm/s zu einer "Quasi-Linie" geformt wird. Durch das hin und her Bewegen des Laserstrahls entstehen somit auch bei einem gepulsten Laser nicht einzelne Krater, sondern eine vertiefte Fläche oder eine Rinne.

Der Polierlaser kann mit einer mittleren Laserleistung von 1 bis 500 W vorzugsweise von etwa 100 bis 300 W betrieben werden. Vorteilhaft ist es, wenn mit dem Polierlaser weniger als 30 und bevorzugt nur 1 bis 10 Überfahrten durchgeführt werden, um die Optik zu polieren.

Der Polierlaser kann auch mit einer Linienlänge betrieben werden, die mindestens so lang wie eine Erstreckung der zu polierenden Fläche ist. Der Laserstrahl wird somit als Linie über die zu polierende Fläche geführt und dabei wird darauf geachtet, dass dabei die gesamte Fläche vom Laserstrahl erfasst wird.

Die der Erfindung zugrunde liegende Aufgabe wird auch von einer Linse gelöst, die in einem Bereich eine um 1 % geringere Dichte aufweist als in einem anderen Bereich der Linse. Ein derartiger Dichtegradient führt zu einem speziellen Brechungsverhalten, wodurch die Linse nicht nur durch ihre Form sondern insbesondere auch durch ihre Dichte in unterschiedlichen Bereichen eine unterschiedliche Lichtbrechung hervorruft. Vorteilhaft ist es, wenn der Unterschied bei 2 bis 5 % oder sogar darüber wie beispielsweise über 10 % liegt.

Vorteilhaft ist es weiterhin, wenn die Linse einen Oberflächenbereich und einen Kernbereich aufweist und die Dichte im Oberflächenbereich höher ist als im Kernbereich. Kumulativ oder alternativ kann die Linse einen kreisförmigen Querschnitt haben und einen radialen Dichtegradienten.

Je nach Einsatzzweck kann es vorteilhaft sein, wenn die Linse weniger als 5 % und vorzugsweise weniger als 1 % der auftreffenden Strahlung reflektiert.

Die Ausführungen zu den Parametern des Lasers beim Abtragen und Polieren sind jede für sich auch unabhängig von der im Patentanspruch 1 angegebenen Pulsdauer und den zuvor genannten weiteren Merkmalen erfindungswesentlich.

Erfindungsgemäße Ausführungsbeispiele sind in der Zeichnung dargestellt und werden im Folgenden beschrieben. Es zeigt
- Figur 1: schematisch einen Rohling zur Herstellung einer Linse,
- Figur 2: schematisch den Rohling während der Laserbearbeitung,
- Figur 3: schematisch den bearbeiteten Rohling nach der Laserbearbeitung,
- Figur 4: schematisch das Auftreffen eines Laserstrahls auf einer Linsenoberfläche,
- Figur 5: schematisch die Schmelze und den erzeugten Dampf nach dem Auftreffen des Laserstrahls,
- Figur 6: schematisch das Ausdampfen des erzeugten Dampfes,
- Figur 7: schematisch einen auf der Linsenoberfläche erzeugten Krater,
- Figur 8: schematisch das Glätten einer Linsenoberfläche mit einem Laserstrahl,
- Figur 9: schematisch die Oberfläche eine unbehandelten Linsenrohlings,
- Figur 10: schematisch die Linsenoberfläche nach dem Laserabtrag,
- Figur 11: schematisch die Linsenoberfläche nach dem Polieren,
- Figur 12: schematisch den Vorschub eines Lasers während der Bearbeitung,
- Figur 13: schematisch die Abhängigkeit der Vorschubgeschwindigkeit von der Laserleistung,
- Figur 14: schematisch eine Draufsicht auf eine Linse mit Dichtegradient,
- Figur 15: schematisch einen Schnitt durch die in Figur 14 gezeigte Linse,
- Figur 16: schematisch die Variation der Impulsintensität über der Zeit,
- Figur 17: schematisch die örtliche Variation der Impulsintensität,
- Figur 18: schematisch einen Impuls mit zentraler Intensitätssenke,
- Figur 19: schematisch die Ausrichtung des Laserstrahls relativ zur Linse,
- Figur 20: schematisch die Laserbearbeitung im Inneren der Linse,
- Figur 21: schematisch unterschiedlich beabstandete Abtragskrater auf der Linsenoberfläche,
- Figur 22: schematisch eine Linse mit einer erhöhten Dichte im Inneren der Linse,
- Figur 23: schematisch eine Linse mit einer erhöhten Dichte an der Oberfläche der Linse,
- Figur 24: schematisch eine Draufsicht auf die in Figur 23 gezeigte Linse,
- Figur 25: schematisch eine Linse mit einer erhöhten Dichte im radial äußeren Bereich der Linse,
- Figur 26: schematisch eine Draufsicht auf die in Figur 25 gezeigte Linse,
- Figur 27: schematisch eine Linse mit in radialer Richtung wechselnder Dichte und
- Figur 28: schematisch eine Draufsicht auf die in Figur 27.

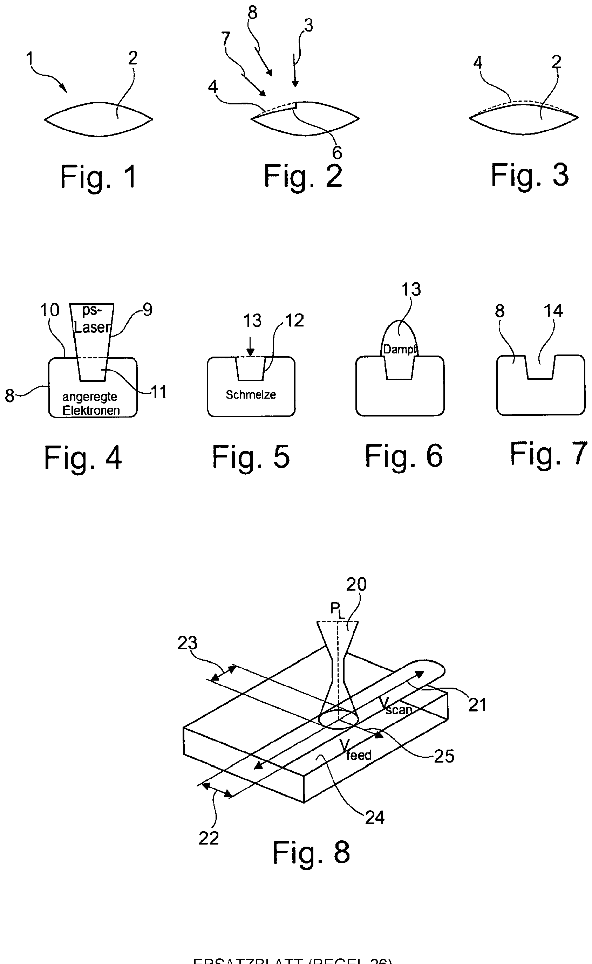

Die Figur 1 zeigt als transmitive Optik 1 einen Linsenrohling 2. Die Figur 2 zeigt, wie dieser Rohling 2 mittels eines Abtragslasers 3 bearbeitet wird. Dabei wurde in dem in Figur 2 gezeigten Beispiel bereits auf der linken Seite der Linse 2 der angedeutete Materialabtrag 4 mit dem Laser 3 erzielt. Nach dem Materialabtrag wird mit der Messeinrichtung 5 die Form 6 der Linse 2 im Bereich der bearbeiteten Oberfläche gemessen. Dies ermöglicht es, auf der Grundlage der Messwerte vorzugsweise noch während der Bearbeitung auf die Art des Impulses des Lasers 3 einzuwirken. Außerdem wird bereits während der Bearbeitung mit dem Pyrometer 7 die Prozesstemperatur überwacht. Auch die Prozesstemperatur kann durch eine Einwirkung auf die Art des Laserstrahls des Lasers 3 beeinflusst und gegebenenfalls sogar geregelt werden.

Nach dem Abtrag hat der Rohling 2 die in Figur 3 gezeigte Form mit einem verringerten Volumen, das auf den Materialabtrag 4 zurückzuführen ist.

Der Rohling ist ein Kunststoff und im vorliegenden Fall ein Acrylat 8. Dieser Rohling kann auch andere Materialien, wie beispielsweise andere Kunststoffe oder Glas, aufweisen. Die zu überarbeitende Oberfläche des Rohlings ist jedoch aus Kunststoff. Die Figur 4 zeigt, wie der Laserstrahl 9 auf die Oberfläche 10 des Acrylats 8 auftrifft und dabei schalenförmig im Bereich 11 in das Acrylat eindringt. Die Pulsdauer des Abtragslasers liegt bei etwa 100 Femtosekunden und dadurch wird im Bereich 11 das Acrylat verdampft. Dabei entsteht ein schalenförmiger Bereich 12 einer Acrylatschmelze und innerhalb dieses schalenförmigen Bereiches 12 ein Bereich 13 aus Dampf.

Die Figur 6 zeigt, wie sich die Schmelze 12 wieder verfestigt und sich der Dampf 13 verflüchtigt. Dadurch bleibt am Ende des Vorgangs der in Figur 7 gezeigte Krater 14 im Acrylatbereich 8.

Durch die Anordnung mehrerer derartiger Krater eng beieinander wird ein flächiger Materialabtrag 4 erzielt. Die dabei erzeugte Oberflächenstruktur ist durch das Aneinanderreihen der Krater rau. Durch eine Minimierung der Kratertiefe und eine Minimierung der Abstände zwischen den Kratern kann die Rauigkeit auf der Kunststoffoberfläche verringert werden.

Vorteilhaft für das Glätten der Oberfläche ist es, wenn die Laserintensität minimiert und/oder die Auftrittsfläche des Lasers auf der zu bearbeitenden Oberfläche vergrößert wird, sodass nur noch Material angeschmolzen wird und möglichst kein Material verdampft. Dazu wird in der Regel ein Polierlaser 20verwendet, der scannend längs der Linie 21 mit einer Scangeschwindigkeit (V_{Scan}) und einer Auftrittsbreite 22, 23 über die Oberfläche 24 geführt wird. Dabei wird der Polierlaser 20 mit einer Vorschubgeschwindigkeit (V_{feed}) in Richtung des Pfeiles 25 senkrecht zur Linie 21 vorwärts bewegt.

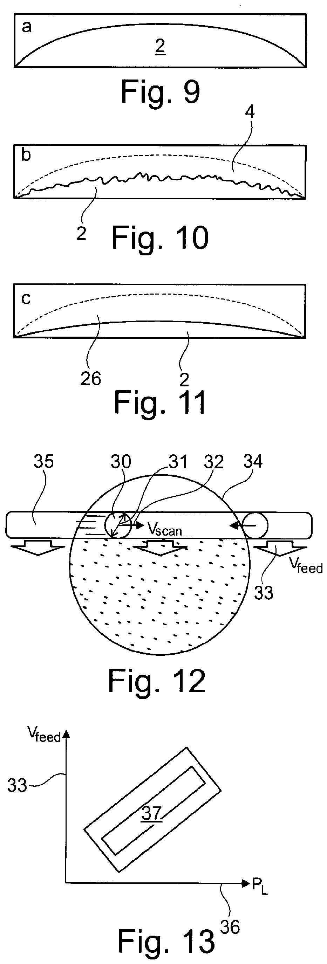

Dies führt dazu, dass, wie in den Figuren 9 bis 11 gezeigt, zunächst der Rohling 2 mit dem Abtragslaser bearbeitet wird, um einen Materialabtrag 4 zu erzielen, wodurch eine raue Oberfläche des Rohlings 2 entsteht. Durch das anschließende Laserpolieren entsteht die in Figur 11 gezeigte glatte Oberfläche 26 des Rohlings 2.

Im Ausführungsbeispiel wird an einer Intraokularlinse 2 aus der in Figur 9 gezeigten Ausgangsform mittels selektivem Materialabtrag die in Figur 10 gezeigte Materialoberfläche erzeugt und anschließend wird diese Materialoberfläche durch Laserpolieren geglättet bis sie transparent ist. Dabei entsteht die in Figur 11 gezeigte Materialoberfläche.

Während des Materialabtrags 4 mit dem Abtragslaser 3 wird darauf geachtet, dass durch die Einwirkung von ultrakurz gepulster Laserstrahlung von etwa 100 oder 200 Femtosekunden nur an der Auftrittsstelle des Lasers auf der Oberfläche ohne thermische Schädigung des umliegenden Materials ein selektiv örtlich begrenzter Materialabtrag erzielt wird. Im Ausführungsbeispiel wird eine Laserwellenlänge von 343 nm verwendet, sodass die Laserstrahlung durch die kleine optische Eindringtiefe dieser Laserwellenlänge im Acrylat oberflächennah absorbiert wird.

Durch einen Vergleich der Ausgangsform und der Zielform der Oberfläche werden die nötige Abtragstiefe und damit die nötige Anzahl der Laserpulse an jeder Stelle der Oberfläche bestimmt. Damit kann ohne Veränderung der Art der Laserstrahlung durch die Anzahl der Laserimpulse pro Fläche der Materialabtrag 4 festgelegt werden. Dabei kann der Laserstahl 30, insbesondere für den Lasermaterialabtrag, mäanderförmig über die zu bearbeitende Oberfläche geführt werden. Auf der Basis der errechneten Anzahl der Laserpulse pro Fläche wird der Laser während des Überfahrens der zu bearbeitenden Fläche ein- und ausgeschaltet.

In dem in Figur 12 gezeigten Ausführungsbeispiel werden ein Strahldurchmesser 31 der Laserstrahlung an der Materialoberfläche von ca. 20 µm, eine Repetitionsrate von 100 kHz und eine Scangeschwindigkeit 32 von 1000 mm/s verwendet. Dadurch entsteht eine Vorschubgeschwindigkeit 33 (V_{feed}), mit der der Laser 30 über die Linse 34 geführt wird.

Für das anschließende Laserpolieren wird ein Laser mit einer Wellenlänge von 10,6 µm verwendet, da auch diese Wellenlänge oberflächennah im Material absorbiert wird. Der Laser wird kontinuierlich betrieben und die Laserleistung liegt im Bereich von 50 bis 100 Watt. Dies führt dazu, dass während des Laserpolierens durch die Einwirkung der Laserstrahlung die Materialoberfläche geschmolzen wird und dann durch die Oberflächenspannung geglättet wird, bevor sie wieder erstarrt.

In dem in Figur 12 gezeigten Ausführungsbeispiel werden 20 Iterationen (Anzahl der Überfahrten) durchgeführt, um die Oberfläche schrittweise zu polieren, wobei jede Iteration die Oberflächenrauheit verringert, bis die Zielrauheit erreicht ist. Zwischen den Iterationen ist eine Pause von 20 Sekunden vorgesehen, um ein Überhitzen der Probe zu verhindern.

Die in Figur 12 gezeigte Verarbeitungsstrategie für die Iteration zeichnet sich dadurch aus, dass eine bidirektionale Scanstrategie mit einer Scangeschwindigkeit von 5000 mm/s verwendet wird und dadurch ein Quasilinienfokus erzeugt wird. Dieser Quasilinienfokus 35 wird mit der Vorschubgeschwindigkeit 33 von 30 bis 40 mm/s über die zu polierende Oberfläche der Linse 34 geführt. Der Strahldurchmesser 31 am Werkstück liegt in diesem Ausführungsbeispiel bei 6 mm. Vorzugsweise wird auch eine Temperaturregelung verwendet, um die Stabilität des Laserpolierens noch zu verbessern.

Bei höherer mittlerer Laserleistung ist eine höhere Vorschubgeschwindigkeit zu verwenden und bei einer verringerten mittleren Laserleistung wird die Vorschubgeschwindigkeit herabgesetzt. Dieser Prozess ist somit skalierbar. Die Abhängigkeit zwischen Vorschubgeschwindigkeit 33 und mittlerer Laserleistung 36 ist in Figur 13 gezeigt. Dabei ergibt sich der schraffierte bevorzugte Arbeitsbereich 37.

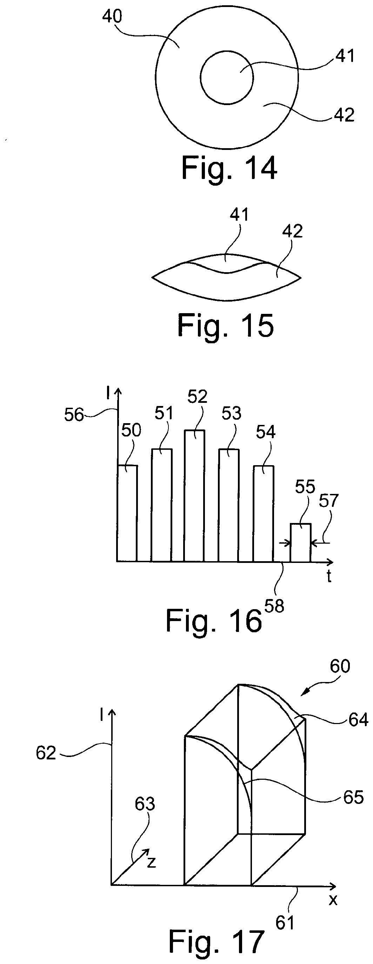

Die Figur 14 zeigt einen speziellen Rohling 40, der durch Spritzgießen hergestellt ist. Dieser Rohling hat als Folge des Spritzgussverfahrens einen Dichtegradienten. Hierbei ist der mittlere Bereich 41 mit einer höheren Dichte ausgebildet als der Randbereich 42. Dieser Dichtegradient kann bei Spritzgießen durch die Druckverhältnisse während des Spritzvorgangs oder auch durch ein Mehrkomponentenspritzgießen erzeugt werden, bei dem verschiedene Kunststoffe verwendet werden. Insbesondere auch bei einer additiven Fertigung aus einem pulverförmigen, flüssigen oder gasförmigen Material kann auf einfache Art und Weise ein Rohling mit einem Dichtegradienten oder aus unterschiedlichen Materialien hergestellt werden. Dieser Dichtegradient führt zu einer speziellen Brechung des Lichtes an der Linse 40. Damit die unterschiedliche Dichte des Rohlings 40 den Vorgang des Materialabtrags und des Polierens nicht beeinträchtigt, kann auch im Inneren der Linse 40 ein Bereich mit anderer Dichte vorgesehen werden, während die zu bearbeitende Oberfläche mit dem für die Bearbeitung relevanten Oberflächenbereich eine einheitliche Dichte aufweist.

Vorteilhaft ist es, wenn die Pulsenergie während des Abtrags und/oder während des Polierens variiert wird. Die Figur 16 zeigt hierzu die Intensität unterschiedlicher zeitlich aufeinander folgender Impulse 50 bis 55, die eine unterschiedliche Intensität 56 aber die gleiche Pulsdauer 57 (nur exemplarisch beziffert) aufweisen. Somit schwankt die Intensität 56 der Impulse 50 bis 55 über der Zeit 58. Entsprechend kann auch die Pulsdauer 57 der einzelnen Pulse 50 bis 55 variieren, während die Pulsintensität konstant bleibt. Letztlich können auch über der Zeit die Intensität 56 und die Pulsdauer 57 variiert und vorzugweise geregelt werden, um optimal auf den Abtrags- oder Poliervorgang einzuwirken und eine schnelle Bearbeitung ohne Überhitzung zu erzielen.

Die örtliche Intensitätsverteilung eines Pulses 60 auf den räumlichen Achsen 61und 63 ist beispielhaft in Figur 17 gezeigt. Dieser Puls zeigt auf der linken Seite eine örtlich begrenzte höhere Pulsenergie 62 als auf der rechten Seite. Dabei kann der Puls beispielsweise längs der Oberfläche 64 langsam abfallen oder längs der gebogenen Oberfläche 65 stark abfallen, sodass die rechte Seite des Pulses eine deutlich geringere Energiekonzentration als dessen linke Seite aufweist. Dies ermöglicht es, beispielsweise bei einem über eine Fläche bewegten Laserstrahl, die während der Zeit auf einen Flächenbereich eingebrachte Strahlungsintensität zu variieren.

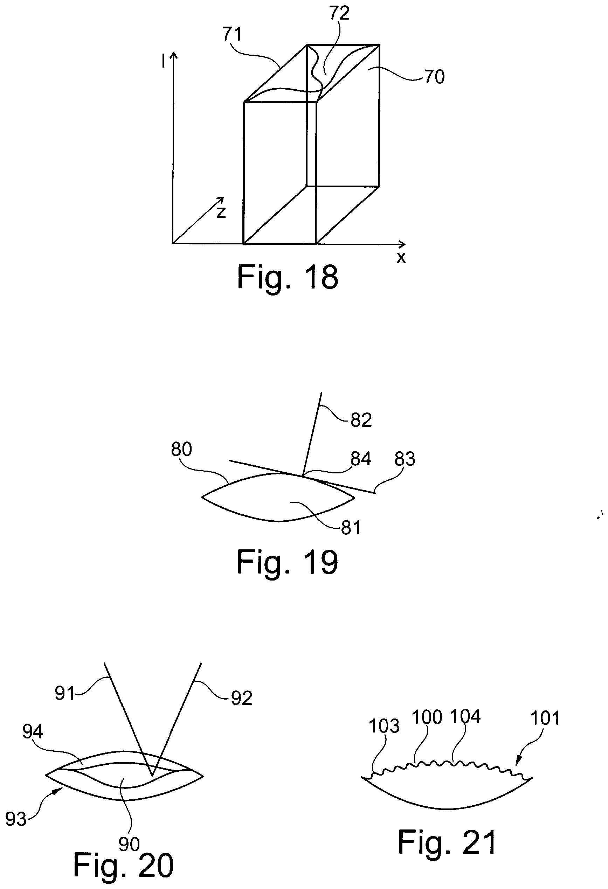

Die Figur 18 zeigt eine spezielle örtliche Energieverteilung eines Pulses 70, bei dem im Randbereich 71 des Pulses 70 eine höhere Energie vorliegt als im zentralen mittleren Bereich 72. Dies führt dazu, dass bei dem Auftreffen des Pulses auf die Oberfläche einer Linse im Randbereich des entstehenden Kraters eine höhere Energie aufgebracht wird als im mittleren Bereich des Kraters. Der Krater erhält daher weniger eine Schüsselform als vielmehr eine Rechteckform, sodass mehrere aneinander gesetzte Krater eine annähernd plane Oberfläche bilden.

Um dies zu optimieren wird vorgeschlagen, dass während der Bearbeitung die Pulsenergieverteilung quer zur Strahlungsrichtung variiert wird.

Um eine homogene Bearbeitung einer linsenförmigen Oberfläche 80 einer Linse 81 zu erzielen, ist es vorteilhaft, wenn der Laserstrahl 82 im Wesentlichen senkrecht zu einer Tangente 83 im Schnittpunkt 84 von Laserstrahl 82 und der Linse 81 gehalten wird. Dies kann dadurch erreicht werden, dass die Ausrichtung des Laserstrahls während der Bearbeitung variiert wird und die Position der Linse 81 konstant gehalten wird oder dadurch, dass die Ausrichtung der Linse 81 relativ zum Laserstrahl 82 geändert wird, indem die Linse 81 während der Bearbeitung bewegt wird. Selbstverständlich können auch Linse und Laser bewegt werden, um eine möglichst senkrechte Ausrichtung des Laserstrahls 82 zur Normalen 83 auf der Oberfläche der Linse zu erzielen. Außerdem kann an Stelle einer Bewegung des Lasers auch der Laserstrahl mit einem Spiegel so ausgerichtet werden, dass er möglichst senkrecht auf die Linsenoberfläche trifft.

Wie am Beispiel der in den Figuren 14 und 15 gezeigten Linse kann die Dichte der Linse durch die Materialwahl oder Materialbearbeitung des Rohlings variiert werden. Die Dichte kann jedoch auch während der Bearbeitung durch den Materialabtrag und/oder das Polieren verändert werden. Dies ermöglicht es, durch die Art des Laserstrahls örtlich begrenzt und als Gradient an der Linsenoberfläche unterschiedliche Dichten vorzusehen. Dabei kann die Dichte an der Materialoberfläche derart gesteigert werden, dass durch den veränderten Brechungsindex Reflexe verhindert werden. Die Dichte kann jedoch mit dem Laserstrahl oder mittels mehrerer Laserstrahlen 91, 92 auch im Inneren 90 einer Linse 93 so verändert werden, dass sich die Lichtbrechung der fertigen Linse nicht als Folge der Oberflächenform der Linse, sondern auch als Folge eines Dichtegradienten im Oberflächenbereich 94 der Linse 93 und/oder im inneren Bereich 90 der Linse 93 ergibt.

Die Anordnung der Abtragskrater 100 auf der Oberfläche 101 einer Linse 102 ist in Figur 21 gezeigt. Dabei sind die Abtragskrater 100 im Randbereich 103 weiter beabstandet als im zentralen Bereich 104. Dies ist nur ein Beispiel, um zu zeigen, wie auch durch die Anzahl der Krater pro Fläche die Art der Bearbeitung der Oberfläche variiert werden kann.

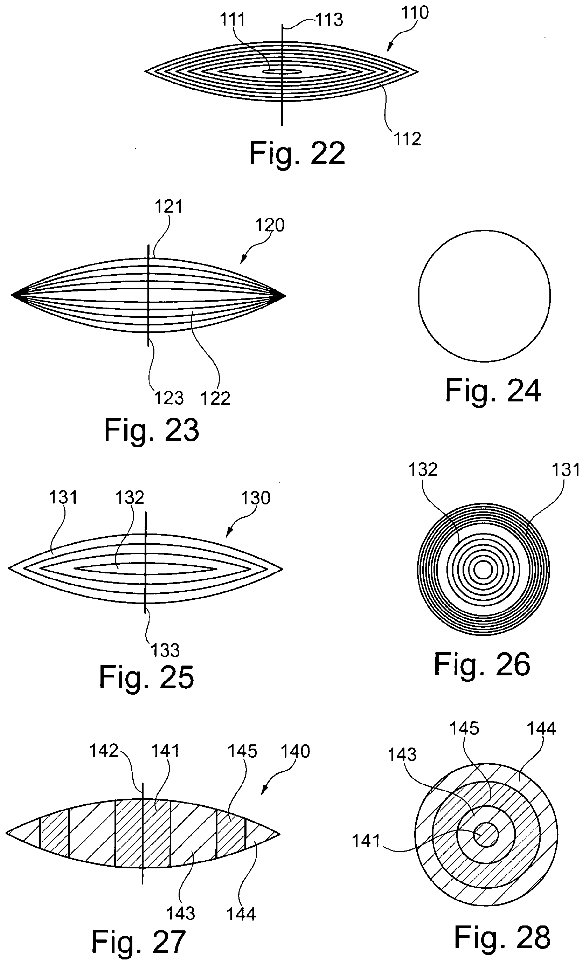

Die in Figur 22 gezeigte Linse 110 hat einen zentralen mittleren Bereich 111, der eine größere Dichte aufweist als der radial äußere Bereich 112.

Die umgekehrte Dichteverteilung wurde bei der in Figur 23 gezeigten Linse 120 realisiert. Dort ist ein äußerer Bereich grafisch dunkler dargestellt, um auf die höhere Dichte hinzuweisen, während der innere Bereich 122 heller dargestellt ist, um auf die verringerte Dichte hinzuweisen.

Bei der in Figur 24 gezeigten Draufsicht erkennt man somit eine einheitliche Dichte, sofern nur die sichtbare Oberfläche betrachtet wird. In beiden Ausführungsbeispielen liegt somit ein Dichtegradient in Richtung der optischen Achse 113 bzw. 123 vor.

Die Figur 25 zeigt eine Linse 130 mit einem radialen Dichtegradienten. Im Bereich der optischen Achse 133 liegt ein Bereich 132 mit geringerer Dichte als im radial weiter außen liegenden Bereich 131. Daher zeigt die Draufsicht in Figur 26 einen dunkleren radial äußeren Bereich 131 und einen helleren zentralen Bereich 132 mit geringerer Dichte.

Die Figur 27 zeigt eine Linse 140 mit einem multivokalen Dichtegradienten. Hierbei wechseln sich in radialer Richtung von einem zentralen mittleren Bereich 141 an der optischen Achse 142 Bereiche 143 und 144 mit geringerer optischer Dichte ab, zwischen denen ein Bereich 145 mit höherer optischer Dichte liegt.

Die Figur 28 zeigt in der Draufsicht, dass die Bereiche höherer und niedrigerer optischer Dichte ringförmig ausgebildet sind.

Bei allen gezeigten Ausführungsbeispielen kann die optische Dichte als Gradient in eine veränderte optische Dichte übergehen und alternativ können Bereiche unterschiedlicher optischer Dichte klar abgegrenzt aneinander liegen. Dabei kann mit der variierenden optischen Dichte auf das Brechungsverhalten der Linse beim Durchtritt eines Lichtstrahls und dessen Ablenkung eingewirkt werden. Alternativ oder kumulativ kann auch auf die Reflexionseigenschaften, insbesondere an der Grenzfläche der Linsenoberfläche über deren Dichte und die in der Regel damit einhergehende Härte eingewirkt werden.

## Patentansprüche

1. Verfahren zur Herstellung einer transmitiven Optik (1) aus einem Rohling (2) aus Kunststoff, wobei an dem Rohling (2) mit einem Abtragslaser (3) ein Materialabtrag (4) erzielt wird, wobei die Pulsdauer des Abtragslasers (3) kleiner 1 ns ist, ***dadurch gekennzeichnet, dass*** die Pulsenergie des Abtragslasers bei 0,1 µJ bis 10 µJ liegt, der Fokusdurchmesser des Abtragslasers zwischen 5 und 50 µm liegt, die Scangeschwindigkeit des Abtragslaser bei 500 bis 5000 mm/s liegt und der Abtragslaser einen Materialabtrag von 0,02 µm bis 5 µm pro Puls bewirkt.

2. Verfahren nach Anspruch 1, ***dadurch gekennzeichnet, dass*** der mit dem Abtragslaser (3) vorbehandelte Rohling (2) mit einem Polierlaser (20) weiterbearbeitet wird.

3. Verfahren nach einem der vorhergehenden Ansprüche, ***dadurch gekennzeichnet, dass*** ein im Querschnitt kreisrunder Rohling (40) verwendet wird, der zur Mitte (41) hin eine andere optische Dichte aufweist als zum Rand (42) hin.

4. Verfahren nach einem der vorhergehenden Ansprüche, ***dadurch gekennzeichnet, dass*** während der Bearbeitung die Prozesstemperatur mit einem Pyrometer (7) überwacht und/oder geregelt wird.

5. Verfahren nach einem der vorhergehenden Ansprüche, ***dadurch gekennzeichnet, dass*** der Rohling auf einer Seite symmetrisch geformt ist und auf einer anderen Seite asymmetrisch oder als Freiform bearbeitet wird.

6. Verfahren nach einem der vorhergehenden Ansprüche, ***dadurch gekennzeichnet, dass*** zunächst ein Auge eines Patienten vermessen wird und damit ein Datensatz erzeugt wird und anschließend auf der Grundlage der Daten dieses Datensatzes der Abtragslaser und/oder der Polierlaser gesteuert wird.

7. Verfahren nach einem der vorhergehenden Ansprüche, ***dadurch gekennzeichnet, dass*** mit der Laserstrahlung das Material des Rohlings derart verändert wird, dass die fertige Linse einen optischen Dichtegradienten aufweist.

8. Verfahren nach einem der vorhergehenden Ansprüche, ***dadurch gekennzeichnet, dass*** die Optik eine Intraokularlinse (IOL) ist.

9. Verfahren nach einem der vorhergehenden Ansprüche, ***dadurch gekennzeichnet, dass*** der Abtragslaser so betrieben wird, dass er einen Materialabtrag von von 0,02 µm bis 0,5 µm bewirkt.

10. Verfahren nach einem der vorhergehenden Ansprüche, ***dadurch gekennzeichnet, dass*** der Abtragslaser mit einer Laserwellenlänge von kleiner 400 nm, wie insbesondere zwischen 193 nm und 370 nm betrieben wird.

11. Verfahren nach einem der vorhergehenden Ansprüche, ***dadurch gekennzeichnet, dass*** der Fokusdurchmesser des Abtragslasers bei etwa 20 µm liegt.

12. Verfahren nach einem der vorhergehenden Ansprüche, ***dadurch gekennzeichnet, dass*** die Scangeschwindigkeit des Abtragslasers bei etwa 1000 mm/s liegt.

13. Verfahren nach einem der vorhergehenden Ansprüche, ***dadurch gekennzeichnet, dass*** die Pulsenergie des Abtragslasers bei etwa 1 µJ liegt.

14. Verfahren nach einem der vorhergehenden Ansprüche, ***dadurch gekennzeichnet, dass*** die Repetitionsrate des Abtragslasers bei 5 kHz bis 5000 kHz und vorzugsweise bei 50 kHz bis 200 kHz liegt.

15. Verfahren nach einem der vorhergehenden Ansprüche, ***dadurch gekennzeichnet, dass*** der Polierlaser mit einer Laserwellenlänge im Bereich zwischen 1 bis 12 µm und besonders bevorzugt zwischen 9 µm und 11 µm betrieben wird.

16. Verfahren nach einem der vorhergehenden Ansprüche, ***dadurch gekennzeichnet, dass*** der Polierlaser kontinuierlich betrieben wird.

17. Verfahren nach einem der vorhergehenden Ansprüche, ***dadurch gekennzeichnet, dass*** der Polierlaser durch eine Scanbewegung mit einer Scangeschwindigkeit von 500 mm/s bis 20000 mm/s zu einer "Quasi-Linie" geformt wird.

18. Verfahren nach einem der vorhergehenden Ansprüche, ***dadurch gekennzeichnet, dass*** mit dem Polierlaser weniger als 30 und bevorzugt 1 bis 10 Überfahrten durchgeführt werden.

## Claims

1. A method for producing a transmissive optics (1) from a blank (2) made of plastics, wherein a material ablation (4) is achieved on the blank (2) with an ablation laser (3), wherein the pulse duration of the ablation laser (3) is less than 1 ns, ***characterized in that*** the pulse energy of the ablation laser is 0.1 µJ to 10 µJ, the focus diameter of the ablation laser is between 5 and 50 µn, the scanning speed of the ablation laser is 500 to 5000 mm/s and the ablation laser effects a material ablation of 0.02 µm per pulse.

2. The method according to claim 1, ***characterized in that*** the blank (2) pretreated with the ablation laser (3) is further processed with a polishing laser (20).

3. The method according to any one of the preceding claims, ***characterized in that*** a blank (40) with a circular cross-section is used, which has an optical density towards the center (41) that differs from that towards the edge (42).

4. The method according to any one of the preceding claims, ***characterized in that*** during processing, the process temperature is monitored and/or controlled with a pyrometer (7).

5. The method according to any one of the preceding claims, ***characterized in that*** the blank is symmetrically formed on one side and is processed asymmetrically or in a free-form manner on another side.

6. The method according to any one of the preceding claims, ***characterized in that*** an eye of a patient is first measured and a data set is generated therefrom, and the ablation laser and/or the polishing laser is subsequently controlled on the basis of the data of this data set.

7. The method according to any one of the preceding claims, ***characterized in that*** with the laser radiation, the material of the blank is altered such that the finished lens has an optical density gradient.

8. The method according to any one of the preceding claims, ***characterized in that*** the optical system is an intraocular lens (IOL).

9. The method according to any one of the preceding claims, ***characterized in that*** the ablation laser is operated such that it effects a material ablation of from 0.02 µm to 0,5 µm.

10. The method according to any one of the preceding claims, ***characterized in that*** the ablation laser is operated with a laser wavelength of less than 400 nm, such as, in particular, between 193 nm and 370 nm.

11. The method according to any one of the preceding claims, ***characterized in that*** the focal diameter of the ablation laser is approximately 20 µm.

12. The method according to any one of the preceding claims, ***characterized in that*** the scanning speed of the ablation laser is approximately 1,000 mm/s.

13. The method according to any one of the preceding claims, ***characterized in that*** the pulse energy of the ablation laser is approximately 1 µJ.

14. The method according to any one of the preceding claims, ***characterized in that*** the repetition rate of the ablation laser is from 5 kHz to 5,000 kHz, and preferably from 50 kHz to 200 kHz.

15. The method according to any one of the preceding claims, ***characterized in that*** the polishing laser is operated with a laser wavelength in the range between 1 and 12 µm, and particularly preferably between 9 µm and 11 µm.

16. The method according to any one of the preceding claims, ***characterized in that*** the polishing laser is operated continuously.

17. The method according to any one of the preceding claims, ***characterized in that*** the polishing laser is formed into a "quasi-line" by means of a scanning movement at a scanning speed of 500 mm/s to 20,000 mm/s.

18. The method according to any one of the preceding claims, ***characterized in that*** less than 30 and preferably 1 to 10 passes are performed with the polishing laser.

## Revendications

1. Procédé, destiné à fabriquer une optique (1) transmissive à partir d'une ébauche (2) en matière plastique, lors duquel une ablation de matière (4) est obtenue sur l'ébauche (2) à l'aide d'un laser d'ablation (3), la durée d'impulsion du laser d'ablation (3) étant inférieure à 1 ns, ***caractérisé en ce que*** l'énergie impulsionnelle du laser d'ablation se situe à de 0,1 µJ à 10 µJ, le diamètre de focalisation du laser d'ablation se situe entre 5 et 50 µm, la vitesse de numérisation du laser d'ablation se situe à de 500 à 5000 mm/s et le laser d'ablation provoque une ablation de matière de 0,02 µm à 5 µm par impulsion.

2. Procédé selon la revendication 1, ***caractérisé en ce que*** l'on usine ultérieurement l'ébauche (2) préalablement usinée avec le laser d'ablation (3) avec un laser de polissage (20).

3. Procédé selon l'une quelconque des revendications précédentes, ***caractérisé en ce que*** l'on utilise une ébauche (40) de section transversale circulaire, qui en direction du centre (41) fait preuve d'une densité optique différente de celle en direction du bord (42).

4. Procédé selon l'une quelconque des revendications précédentes, ***caractérisé en ce que*** pendant l'usinage, l'on supervise et / ou l'on régule la température de processus à l'aide d'un pyromètre (7).

5. Procédé selon l'une quelconque des revendications précédentes, ***caractérisé en ce que*** sur une face, l'ébauche est façonnée de manière symétrique et sur une autre face, on l'usine de forme asymétrique ou de forme libre.

6. Procédé selon l'une quelconque des revendications précédentes, ***caractérisé en ce que*** dans un premier temps, l'on mesure un oeil d'un patient et l'on génère ainsi un jeu de données et par la suite, sur la base des données dudit jeu de données, on commande le laser d'ablation et / ou le laser de polissage.

7. Procédé selon l'une quelconque des revendications précédentes, ***caractérisé en ce qu**'*à l'aide du faisceau laser, l'on fait varier la matière de l'ébauche de telle sorte que la lentille finie fasse preuve d'un gradient de densité optique.

8. Procédé selon l'une quelconque des revendications précédentes, ***caractérisé en ce que*** l'optique est une lentille intraoculaire (LIO).

9. Procédé selon l'une quelconque des revendications précédentes, ***caractérisé en ce que*** l'on fait fonctionner le laser d'ablation de sorte qu'il provoque une ablation de matière de 0,02 µm à 0,5 µm.

10. Procédé selon l'une quelconque des revendications précédentes, ***caractérisé en ce que*** l'on fait fonctionner le laser d'ablation avec une longueur d'onde d'émission de faisceau laser inférieure à 400 nm, telle que comprise notamment entre 193 nm et 370 nm.

11. Procédé selon l'une quelconque des revendications précédentes, ***caractérisé en ce que*** le diamètre de focalisation du laser d'ablation se situe à environ 20 µm.

12. Procédé selon l'une quelconque des revendications précédentes, ***caractérisé en ce que*** la vitesse de numérisation du laser d'ablation se situe à environ 1000 mm/s.

13. Procédé selon l'une quelconque des revendications précédentes, ***caractérisé en ce que*** l'énergie impulsionnelle du laser d'ablation se situe à environ 1 µJ.

14. Procédé selon l'une quelconque des revendications précédentes, ***caractérisé en ce que*** la fréquence de répétition du laser d'ablation se situe à de 5 kHz à 5000 kHz et de préférence à de 50 kHz à 200 kHz.

15. Procédé selon l'une quelconque des revendications précédentes, ***caractérisé en ce que*** l'on fait fonctionner le laser de polissage à une longueur d'onde d'émission de faisceau laser de l'ordre compris entre 1 et 12 µm et de manière particulièrement préférentielle, entre 9 µm et 11 µm.

16. Procédé selon l'une quelconque des revendications précédentes, ***caractérisé en ce que*** l'on fait fonctionner en continu le laser de polissage.

17. Procédé selon l'une quelconque des revendications précédentes, ***caractérisé en ce que*** par un mouvement de balayage à une vitesse de numérisation de 500 mm/s à 20000 mm/s, l'on forme le laser de polissage en une « quasi-ligne ».

18. Procédé selon l'une quelconque des revendications précédentes, ***caractérisé en ce qu'***à l'aide du laser de polissage, l'on procède à moins de 30 et de préférence à de 1 à 10 passages.
